(19) **Europäisches Patentamt / European Patent Office / Office européen des brevets**

(11) **EP 4 560 365 A1**

(12) **EUROPEAN PATENT APPLICATION**
published in accordance with Art. 153(4) EPC

(43) Date of publication:
**28.05.2025 Bulletin 2025/22**

(21) Application number: **23843292.6**

(22) Date of filing: **17.07.2023**

(51) International Patent Classification (IPC):
**G02B 3/10** (2006.01)    **G02B 5/00** (2006.01)

(52) Cooperative Patent Classification (CPC):
**G02B 3/10; G02B 5/00**

(86) International application number:
**PCT/KR2023/010165**

(87) International publication number:
**WO 2024/019443 (25.01.2024 Gazette 2024/04)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **18.07.2022 KR 20220088108**

(71) Applicant: **IUCF-HYU (Industry-University Cooperation Foundation Hanyang University)
Seoul 04763 (KR)**

(72) Inventor: **SONG, Seok Ho
Seoul 06520 (KR)**

(74) Representative: **dompatent von Kreisler Selting Werner -
Partnerschaft von Patent- und Rechtsanwälten mbB
Deichmannhaus am Dom
Bahnhofsvorplatz 1
50667 Köln (DE)**

(54) **MULTIFOCAL LENS HAVING CENTRAL AXICON PORTION**

(57)    The present invention relates to a composite diffractive intraocular lens having one or more optical magnifications or focal lengths, the lens including: a base lens forming a single focal point; a diffractive lens formed on the base lens, provided with a central region and a diffractive region surrounding the central region, and configured to disperse the single focal point into multiple focal points to form multiple focal points; and an axicon portion formed in the central region to protrude in a central axis direction of the base lens such that a depth of focus is expanded between the multiple focal points.

[FIG. 3]

EP 4 560 365 A1

**Description**

[Technical Field]

The present invention relates to a multifocal lens having a central axicon portion, and more particularly to a multifocal lens having a central axicon portion which can obtain an extended depth of focus between focal points created by forming an axicon shape in the central region of a lens element.

[Background Art]

Multifocal or multi-focus diffractive lenses are widely used in optical lenses, especially ophthalmic intraocular lenses, and are widely used for ophthalmic therapeutic purposes to provide high-quality vision for both near and far objects simultaneously.

For example, multifocal intraocular lenses (MF-IOLs) having a surface including one or more triple diffraction patterns and one or more double diffraction patterns have been commercialized such that a double focus diffraction pattern provides nearsighted and hyperopic vision and a triple focus diffraction pattern provides nearsighted, hyperopic, and intermediate vision.

As the technology for manufacturing multifocal diffraction lenses that have been commercialized so far, a method of precisely processing the anterior surface or posterior surface of a single-focus lens to have a surface-relief shape in the form of a Fresnel diffraction lens is being widely used.

However, as the number of focal points of multifocal diffractive lenses increases, the surface-relief shape of the lens to be manufactured gradually becomes more complex, making it very difficult to manufacture MF-IOLs, and the problem of increased vision blurring (blurring, halo, starburst, etc.) due to severe light scattering caused by imperfect manufacturing conditions is emerging.

[Disclosure]

[Technical Problem]

Therefore, the present invention has been made in view of the above problems, and it is one object of the present invention to provide a multifocal lens having the effects of existing diffractive multifocal lenses while allowing the depth of focus between created focal points to be expanded.

[Technical Solution]

In accordance with another aspect of the present invention, provided is a multifocal lens having a central axicon portion, the multifocal lens including: a base lens forming a single focal point; a diffractive lens formed on the base lens, provided with a central region and a diffractive region surrounding the central region, and configured to disperse the single focal point into multiple focal points to form multiple focal points; and an axicon portion formed in the central region to protrude in a central axis direction of the base lens such that a depth of focus is expanded between the multiple focal points.

In addition, in an embodiment of the present invention, the diffractive lens and the axicon portion may be formed on a front or back surface or both surfaces of the base lens.

In addition, in an embodiment of the present invention, a shape of the axicon portion may be modeled as in a mathematical equation below:

<Mathematical equation>

$$z = -\sqrt{A^2 + \frac{r^2}{\tan^2\left(\frac{\theta}{2}\right)}}$$

where z indicates a distance from a vertex axis, which passes through a vertex of an axicon shape and is perpendicular to the central axis, to the axicon shape, A indicates a distance from the vertex axis to the vertex of the axicon shape, r indicates a radius that is a distance from the central axis to the axicon shape, and θ indicates an angle between two generators of the axicon shape.

In addition, in an embodiment of the present invention, an angle (θ) between both generators of the axicon shape may not be 180°.

In addition, in an embodiment of the present invention, extended-depth-of-focus effects may increase as the distance A from the vertex axis to the vertex of the axicon shape increases in a state where the angle (θ) between both the generators of the axicon shape is fixed.

In addition, in an embodiment of the present invention, extended-depth-of-focus effects may increase as the angle (θ) between both the generators of the axicon shape is reduced in a state where the distance A from the vertex axis to the vertex of the axicon shape is fixed.

In addition, in an embodiment of the present invention, the distance A from the vertex axis to the vertex of the axicon shape or the angle (θ) between both the generators of the axicon shape may be set such that extended-depth-of-focus effects increase in a distance between a near-distance focal point and an intermediate-distance focal point in a focal point generated along the axicon shape.

In addition, in an embodiment of the present invention, the multifocal lens may have a concave shape when looking at a reference vertex q from the central axis when a sign of the z value becomes (+).

In addition, in an embodiment of the present invention, the distance A from the vertex axis to the vertex of the axicon shape or the angle ($\theta$) between both the generators of the axicon shape may be set such that extended-depth-of-focus effects increase in a distance between an intermediate-distance focal point and a distant-distance focal point in a focal point generated along the axicon shape when a sign of the z value becomes (+).

In addition, in an embodiment of the present invention, a size of the central region may affect light intensity of an extended depth of focus.

In addition, in an embodiment of the present invention, heights of a plurality of steps in the diffractive region may increase or decrease along a radial direction of a lens element.

In addition, in an embodiment of the present invention, a height of a vertex of an axicon shape formed in the central region may be equal to or smaller than a height of a step formed in a diffractive region.

In addition, in an embodiment of the present invention, the central region and the diffractive region may be attached to each other based on a certain boundary line.

[Advantageous effects]

As apparent above, the central region of a lens element can be manufactured into various axicon shapes to obtain a desired depth of focus expansion effect, and in particular, the depth of focus can be expanded between a near distance and an intermediate distance required when reading a smartphone or a book, etc.

[Description of Drawings]

FIG. 1 illustrates the schematic side view of an existing diffractive multifocal intraocular lens.
FIG. 2 illustrates the schematic top view of an existing diffractive multifocal intraocular lens.
FIG. 3 illustrates the schematic side view of a multifocal lens having a central axicon portion according to the present invention.
FIG. 4 illustrates a central region and diffractive region of the multifocal lens having a central axicon portion according to the present invention.
FIG. 5 illustrates the cross-section of the central axicon portion according to the present invention.
FIG. 6 illustrates a surface profile according to the radius of the multifocal lens having a central axicon portion according to the present invention.
FIG. 7 illustrates an example in which three focal points are generated by the multifocal lens having a central axicon portion according to the present invention.
FIG. 8 illustrates a light intensity distribution dependent upon a focal point distance when there is no axicon portion in the central region.
FIG. 9a illustrates radius-dependent axicon heights when $\theta = 179.9°$ and A = 0 $\mu$m in the axicon shape according to the present invention, and FIG. 9b illustrates light intensity distribution dependent upon the focal distance formed by the multifocal lens having the axicon portion according to FIG. 9a.
FIG. 10a illustrates radius-dependent axicon heights when $\theta = 179.9°$ and A = 0.5 $\mu$m in the axicon shape according to the present invention, and FIG. 10b illustrates light intensity distribution dependent upon the focal distance formed by the multifocal lens having the axicon portion according to FIG. 10a.
FIG. 11a illustrates radius-dependent axicon heights when $\theta = 179.9°$ and A = 1.0 $\mu$m in the axicon shape according to the present invention, and FIG. 11b illustrates light intensity distribution dependent upon the focal distance formed by the multifocal lens having the axicon portion according to FIG. 11a.
FIG. 12a illustrates radius-dependent axicon heights when $\theta = 179.8°$ and A = 0 $\mu$m in the axicon shape according to the present invention, and FIG. 12b illustrates light intensity distribution dependent upon the focal distance formed by the multifocal lens having the axicon portion according to FIG. 12a.
FIG. 13a illustrates radius-dependent axicon heights when $\theta = 179.8°$ and A = 0.5 $\mu$m in the axicon shape according to the present invention, and FIG. 13b illustrates light intensity distribution dependent upon the focal distance formed by the multifocal lens having the axicon portion according to FIG. 13a.
FIG. 14a illustrates radius-dependent axicon heights when $\theta = 179.8°$ and A = 1.05 $\mu$m in the axicon shape according to the present invention, and FIG. 14b illustrates light intensity distribution dependent upon the focal distance formed by the multifocal lens having the axicon portion according to FIG. 14a.

[Best Mode]

Hereinafter, the present invention according to a preferred embodiment will be described in detail with reference to the attached drawings. Here, the same symbols are used for the same components, and repetitive descriptions and detailed descriptions of known functions and configurations that may unnecessarily obscure the gist of the invention are omitted. Embodiments of the invention are provided to more completely explain the present invention to those with average knowledge in the art. Therefore, the shapes and sizes of elements in the drawings may be exaggerated for clearer explanation

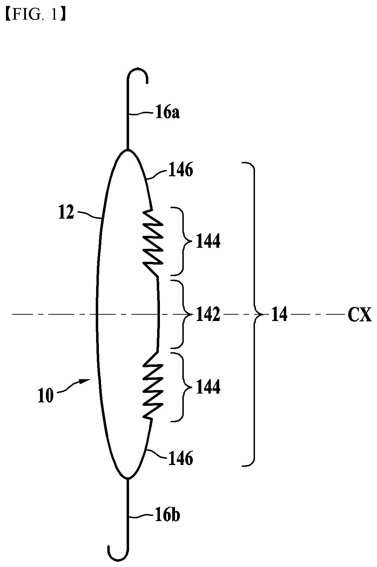

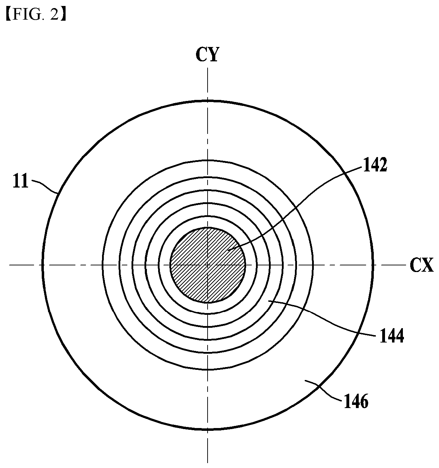

FIG. 1 illustrates the schematic side view of an existing diffractive multifocal intraocular lens, and FIG. 2 illustrates the schematic top view of an existing diffractive multifocal intraocular lens.

The present invention has a shape in which the central region of the existing diffractive multifocal intraocular lens has been changed. Of course, it may be applied to existing various lenses having diffractive multifocal in addition to the intraocular lens. Therefore, since the remaining configuration except for the central region of the existing diffractive multifocal intraocular lens as shown in FIGS. 1 and 2 may be applied to the present invention as is, the existing diffractive multifocal intraocular lens is described first.

Referring to FIGS. 1 and 2, the existing diffractive multifocal intraocular lens is composed of a base lens and diffractive lens combined, and is composed of a lens element 10 having a lens surface composed of a front surface 12 and a back surface 11. In FIGS. 1 and 2, the lens element is illustrated as an intraocular lens (IOL), but is not limited thereto, and may be composed of various lenses for various purposes, such as a contact lens, an artificial cornea, or a lamellar implant.

The lens element 10 may be formed of a suitable biocompatible material. For example, it may be formed of a generally known lens material including, but not limited to, soft acrylic, silicone, hydrogel, or other biocompatible polymeric material having a refractive index required for a particular lens application.

Meanwhile, in the present invention, both the surfaces 12 and 11 of the base lens of which the surface of the lens element 10 is not processed may have the same or different basic (radial) profiles. That is, the front and back surfaces of the base lens may have various surface profiles such as a convex-convex profile, a convex-concave profile, a plano-convex profile, etc.

A central region 142 having a constant radius centered around a central axis CX is formed at the center of the back surface of the lens element 10. Here, the central axis CX may be an optical axis. The central region 142 is a region that contributes to forming a refractive focus of the lens element 10, and according to an embodiment, may provide near, intermediate, and far vision together with diffractive foci formed in diffractive regions 144.

The central region 142 may have an aspherical profile or a spherical profile to create a single refractive focus. According to an embodiment, the central region 142 may be further strengthened for improved focal point formation by having different central region curvatures.

Annular diffractive regions 144 are formed around the central region 142. The annular diffractive regions 144 may provide multiple focal points, such as a near-distance focal point, an intermediate-distance focal point, and a distant-distance focal point, by surrounding the central region 142. The diffractive regions 144 may be formed of multiple annular regions using multiple diffraction orders such as 0, +1, -1, etc.

As shown in FIG. 1, the multi-focal diffraction region 144 has grooves having multiple diffraction step heights, and the diffraction steps forming these grooves may be distributed from a height for steering light along a diffraction order related to a near-distance focal point to a height for steering light along a diffraction order related to a distant-distance focal point. Here, the grooves may be formed in various shapes such as a linear, spherical or trigonometric cosine shape, or an aspherical shape. Meanwhile, there is no limitation on the number of grooves forming a diffraction zone.

As shown in FIGS. 1 and 2, the diffractive regions 144 are separated from each other by a plurality of steps. According to an embodiment, the steps of the diffractive regions may have various heights. For example, the heights of the steps may be uniform or non-uniform or may increase (reverse apodizing) or decrease (apodizing) in a radial direction (radial direction) from the center of the lens element 10.

Outer regions 146 may provide a single refractive focus power similar to that in the central region 142, and in this case, may have substantially the same refractive power as that provided by the central region 142. Accordingly, the outer regions 146 may contribute to providing the distant-distance focus vision of light energy incident on the lens element 10 as in the central region 142. In another embodiment, the outer regions 146 may be formed by extending the diffractive regions 144.

Meanwhile, the existing diffractive multifocal intraocular lens include lens supports 16a and 16b. The lens supports 16a and 16b are components that adjust the position of the intraocular lens (IOL) in the patient's eye. The lens supports 16a and 16b may be formed of suitable polymeric materials such as polymethyl methacrylate, polypropylene, etc.

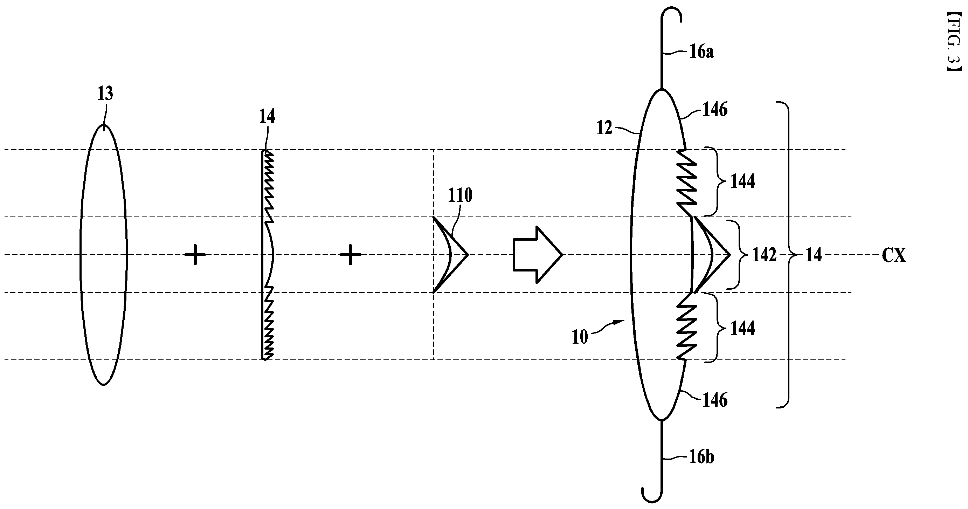

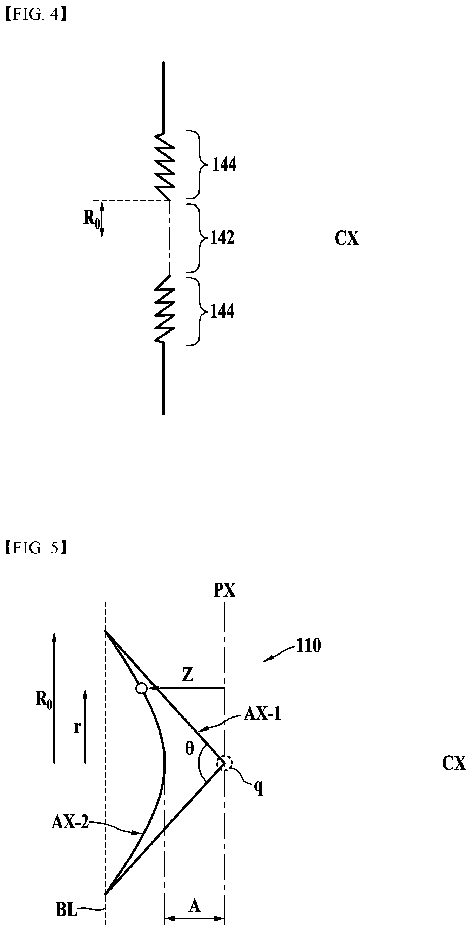

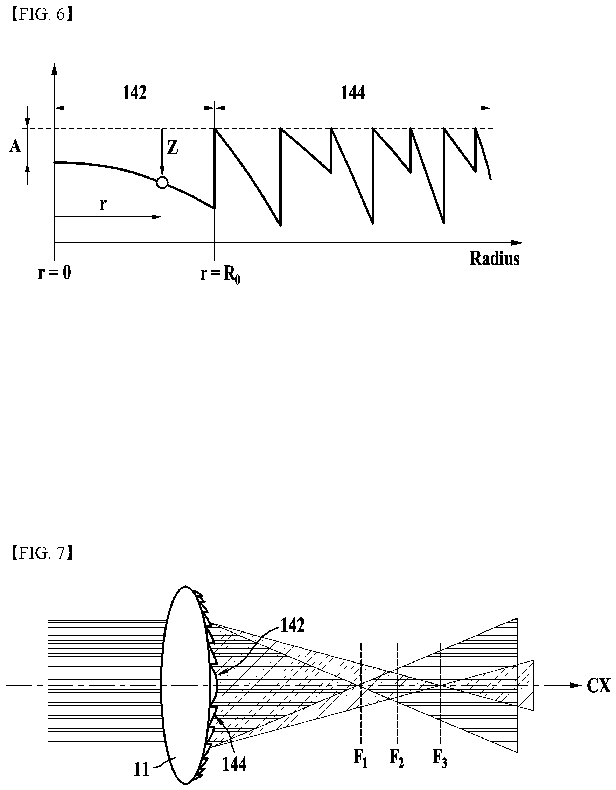

FIG. 3 illustrates the schematic side view of a multifocal lens having a central axicon portion according to the present invention, FIG. 4 illustrates a central region and diffractive region of the multifocal lens having a central axicon portion according to the present invention, FIG. 5 illustrates the cross-section of the central axicon portion according to the present invention, and FIG. 6 illustrates a surface profile according to the radius of the multifocal lens having a central axicon portion according to the present invention.

Referring to FIG. 3, the multifocal lens having a central axicon portion according to an embodiment of the present invention further includes an axicon portion 110 which is to be coupled to the lens element 10, to which the base lens 13 and the diffractive lens 14 are coupled, of the existing diffractive multifocal lens.

That is, the multifocal lens having a central axicon portion according to an embodiment of the present invention is manufactured by coupling the axicon portion 110 to the central region 142 of each of existing diffractive lenses (base lens + diffractive lens) having various shapes.

Referring to FIGS. 3 and 4, the central region 142 is formed in the central portion of a front surface 12 or back surface 11 of the lens element 10. The axicon portion 110 is coupled to the central region 142 of the present invention. The axicon of the axicon portion 110 may have a conical shape. Specifically, the axicon shape has a roughly circular shape with radius R0 along a radial direction about a central axis CX that is an optical axis, and has a shape protruding from the surface of the lens to have a vertex along the central axis direction. In the present invention, the axicon shape may be changed to have a pointed or rounded apex. This is to obtain various extended-depth-of-focus effects depending on the purpose of use, which will be described later.

In an embodiment of the present invention, the diffractive regions 144 are formed in an annular shape to surround the central region 142. Here, since the diffractive regions 144 are in contact with the central region 142, the boundary between the diffractive regions 144 and the central region 142 with respect to the central axis CX becomes the radius $R_0$. As described above, the steps in the diffractive regions 144 may have various heights in a radial direction from the center of the lens element 10.

In another embodiment of the present invention, the central region 142 and the diffractive regions 144 may be configured to be separated from each other.

FIG. 5 illustrates a cross-sectional profile of two axicon shapes formed in the center of the lens element 10. Meanwhile, a baseline BL of the axicon shape shown in FIG. 4 means a line indicating a portion where the radius of the axicon shape is maximum. In an embodiment of the present invention, the baseline BL is the central region of the lens element 10, so the size of the baseline BL is $2R_0$.

Examining the cross-section of a first axicon shape shown in FIG. 4, two generators AX-1 are formed symmetrically about the central axis CX based on a reference vertex q. Here, the generators AX-1 of the first axicon shape have a straight shape, and the angle between the two generators (AX-1) is θ1. The first axicon shape may be said to be an axicon with a pointed apex because the straight-line generators AX-1 meet. Meanwhile, an axis that is perpendicular to the central axis CX and passes through the reference vertex q is called a vertex axis PX.

Examining the cross-section of a second axicon shape shown in FIG. 5, two generators AX-2 are formed symmetrically about the central axis CX based on a vertex. Here, the generators AX-2 of the second axicon shape have a curved shape, and the angle between the two generators AX-2 is θ2. The second axicon shape may be said to be an axicon formed on a shape with a round vertex because the curved generators AX-2 meet.

In the cross-section of the axicon shape, the angle (θ1 or θ2) between the generators (AX-1 or AX-2) is greater than 0° and less than 180°. If the angle between the generators is 0° or 180°, it cannot be called an axicon shape. In addition, examining the cross-section of the axicon shape in an embodiment of the present invention shown in FIG. 5, a distance from the central axis CX to a point where the generator AX-1 or AX-2 meets the baseline BL is $R_0$.

Meanwhile, although FIG. 4 illustrates two axicon shapes, various axicon shapes may be formed according to the following mathematical equation below.

Specifically, the axicon shape according to the present invention may be calculated the following <Mathematical equation>:

<Mathematical equation>

$$z = -\sqrt{A^2 + \frac{r^2}{\tan^2\left(\frac{\theta}{2}\right)}}$$

where z indicates a distance from the vertex axis, which passes through the vertex of the axicon shape and is perpendicular to the central axis, to the axicon shape, A indicates a distance from the vertex axis to the vertex of the axicon shape, r indicates a radius that is a distance from the central axis to the axicon shape, and θ indicates an angle between the two generators of the axicon shape.

Meanwhile, the (-) sign of the z value in the mathematical equation means that it has a convex shape when looking at the reference vertex q from the central axis CX in FIG. 5. According to an embodiment, if the z value in the mathematical equation becomes a + sign, it means that it has a concave shape when looking at the reference vertex q.

Referring to FIG. 5 again, if the A value is 0, an axicon shape having a sharp vertex is formed, and if the A value is greater than 0, an axicon shape having a rounded shape around its A vertex is formed. In the present invention, the height of the axicon shape may be expressed based on the baseline BL or according to the A value. That is, the height of the axicon shape decreases as the A value increases.

Referring to FIG. 6 according to an embodiment of the present invention, when the A value is 0, The vertex height of the axicon shape which is the central region 142 is the same as the step heights of the diffractive regions 144. However, in another embodiment, the vertex height of the axicon shape where A becomes 0 based on the baseline BL may be set in various ways. In this case, it may be different from the step heights of the diffractive regions 144.

According to the present invention, various axicon shapes may be formed by changing the A value and θ value in the mathematical equation described above. When the axicon shape according to the present invention is formed in the center of the lens element 10 in this way, a multifocal intraocular lens having various expanded depths may be formed.

A diffractive multifocal lens may be formed to have a near-distance focal point, an intermediate-distance focal point and a distant-distance focal point. Here, the length at which the size of the focal point is maintained to some extent between the formed focal points is called the depth of focus, and the present invention provides an extended depth of focus to existing diffractive multifocal lenses.

An existing diffractive multifocal lens is related to a technology for obtaining an extended depth of focus between an intermediate distance and a distant distance. However, the focal point distance recently required for reading a smartphone or a book is between a near distance and an intermediate distance, so the need for a multifocal lens having an extended depth of focus at this distance has been raised. The present invention may provide an extended depth of focus, especially at a near-distance to an intermediate-distance.

Meanwhile, if the z value in the mathematical equation becomes a (+) sign as described above, an extended depth of focus may be provided at an intermediate distance to a distant distance. Various expanded depth effects may be obtained by adjusting the distance (A) from the axicon axis to the axicon shape's vertex or the angle ($\theta$) between the two generators of the axicon shape.

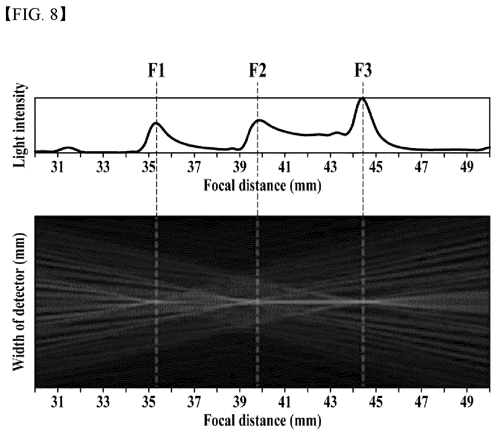

FIG. 7 illustrates an example in which three focal points are generated by the multifocal lens having a central axicon portion according to the present invention, and FIG. 8 illustrates a light intensity distribution dependent upon a focal point distance when there is no axicon portion in the central region.

FIG. 7 illustrates a state in which three focal points F1, F2 and F3 are formed after incident light passes through the multifocal lens having a central axicon portion of the present invention by the central region 142 formed at the center of the lens element 10 and the diffractive regions 144. Here, F1 may be called a near-distance focal point, F2 may be called an intermediate-distance focal point, and F3 may be called a distant-distance focal point. According to an embodiment, various numbers of focal points may be formed depending on the shape of the base lens 13, the diffractive lens 14 or the axicon portion 110 of the present invention, but in this specification, a case where three focal points are formed as shown in FIG. 7 will be described as an example for the convenience of understanding.

FIG. 8 illustrates a case where $\theta = 0°$ and A = 0 $\mu$m which are values that constitute an axicon shape in the mathematical equation described above, and if $\theta = 0°$, it means that the central region is a plane. That is, the axicon shape according to the present invention is not formed. Here, three focal points F1, F2 and F3 are formed as described above. The graph shown on the upper side of FIG. 8 illustrates the light intensity according to the focal distance, and the graph shown on the lower side illustrates the light intensity distribution detected by a detector.

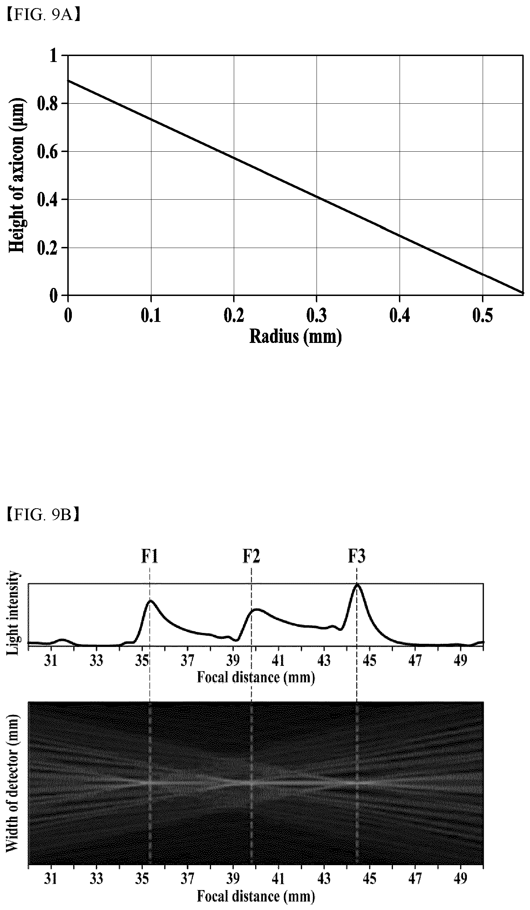

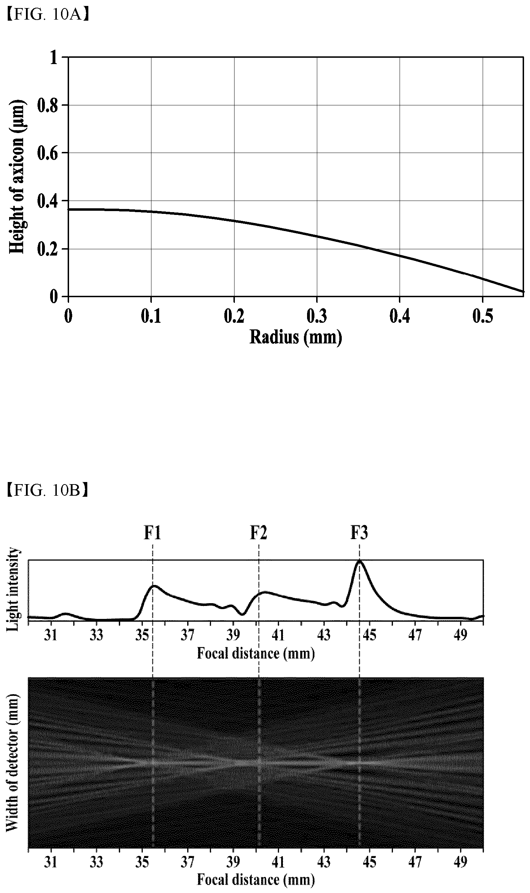

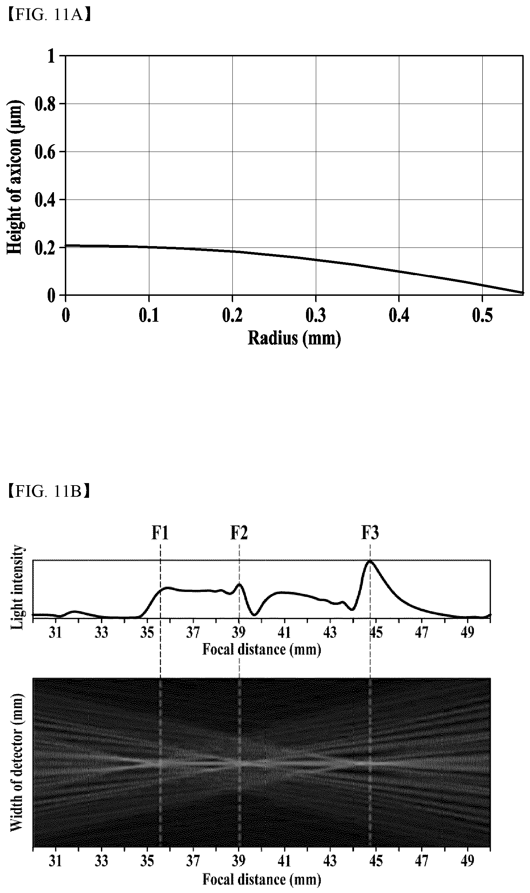

FIG. 9a illustrates radius-dependent axicon heights when $\theta = 179.9°$ and A = 0 $\mu$m in the axicon shape according to the present invention, FIG. 9b illustrates light intensity distribution dependent upon the focal distance formed by the multifocal lens having the axicon portion according to FIG. 9a, FIG. 10a illustrates radius-dependent axicon heights when $\theta = 179.9°$ and A = 0.5 $\mu$m in the axicon shape according to the present invention, FIG. 10b illustrates light intensity distribution dependent upon the focal distance formed by the multifocal lens having the axicon portion according to FIG. 10a, FIG. 11a illustrates radius-dependent axicon heights when $\theta = 179.9°$ and A = 1.0 $\mu$m in the axicon shape according to the present invention, and FIG. 11b illustrates light intensity distribution dependent upon the focal distance formed by the multifocal lens having the axicon portion according to FIG. 11a.

FIGS. 9a to 11b illustrate light intensity distribution dependent upon the axicon height and the focal distance when $\theta$ is fixed to 179.9° and the A value is 0 $\mu$m, 0.5 $\mu$m and 1.0 $\mu$m in the above-described mathematical equation. Referring to FIGS. 9a, 10a and 11a, the height of the axicon shape is about 0.9 $\mu$m when A = 0 $\mu$m, the height of the axicon shape is about 0.4 $\mu$m when A = 0.5 $\mu$m, and the height of the axicon shape is about 0.2 $\mu$m when A = 1.0 $\mu$m.

As shown in FIGS. 9a to 11b, the light intensity between F1 and F2 gradually increases (in FIGS. 9b, 10b and 11b, the brightness between F1 and F2 detected by a detector) as the A value increases (as the axicon height decreases and the curvature increases) when the $\theta$ value is fixed, so the extended-depth-of-focus effects becomes prominent.

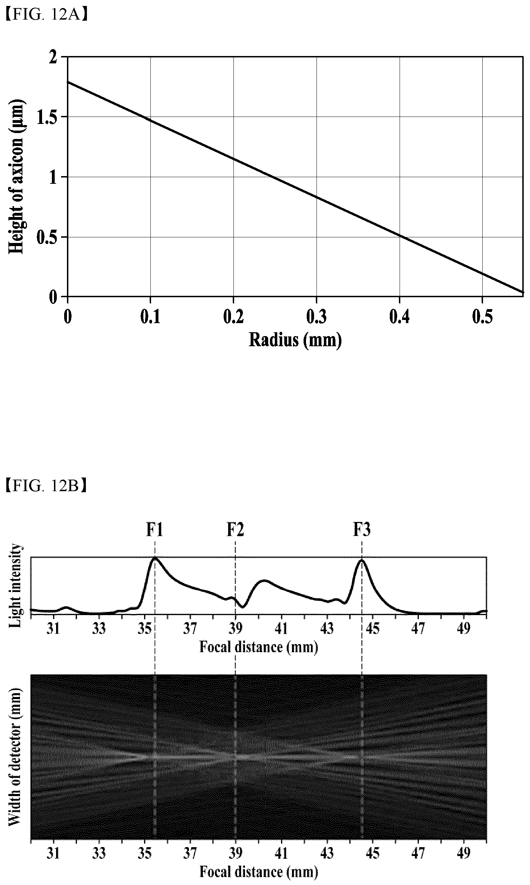

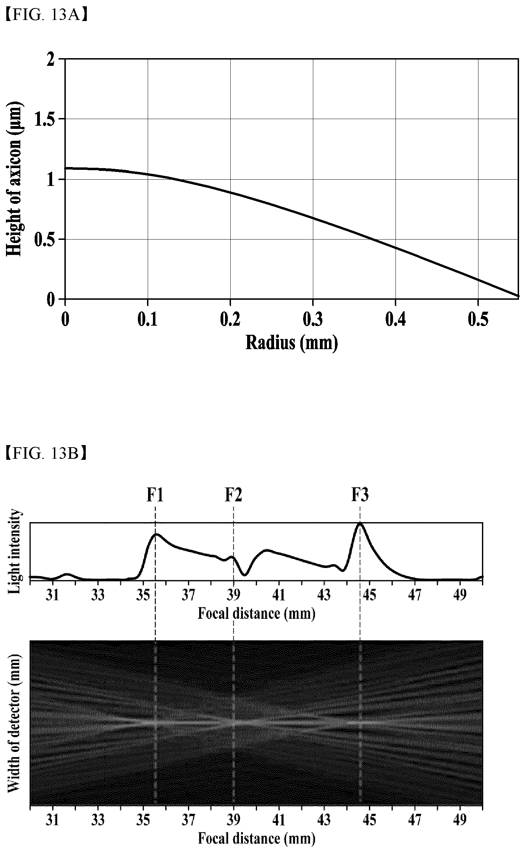

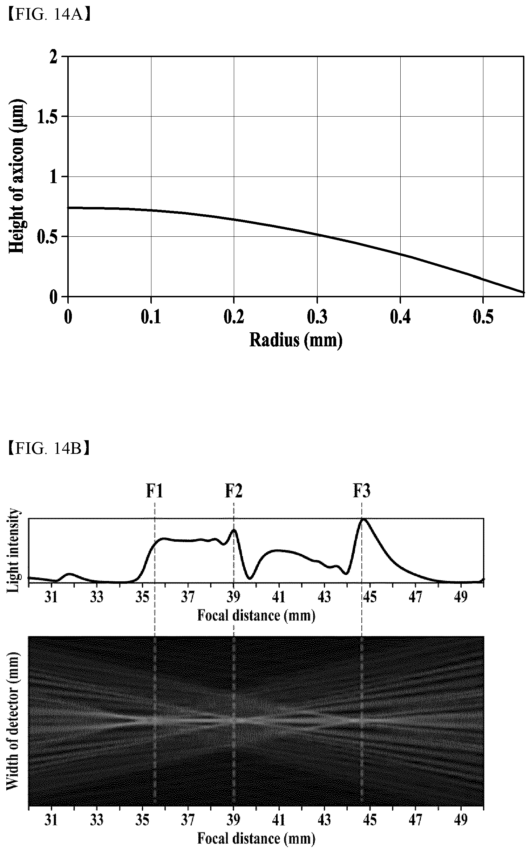

FIG. 12a illustrates radius-dependent axicon heights when $\theta = 179.8°$ and A = 0 $\mu$m in the axicon shape according to the present invention, FIG. 12b illustrates light intensity distribution dependent upon the focal distance formed by the multifocal lens having the axicon portion according to FIG. 12a, FIG. 13a illustrates radius-dependent axicon heights when $\theta = 179.8°$ and A = 0.5 $\mu$m in the axicon shape according to the present invention, FIG. 13b illustrates light intensity distribution dependent upon the focal distance formed by the multifocal lens having the axicon portion according to FIG. 13a, FIG. 14a illustrates radius-dependent axicon heights when $\theta = 179.8°$ and A = 1.05 $\mu$m in the axicon shape according to the present invention, and FIG. 14b illustrates light intensity distribution dependent upon the focal distance formed by the multifocal lens having the axicon portion according to FIG. 14a.

As shown in FIGS. 12a to 14b, the light intensity between F1 and F2 gradually increases (in FIGS. 12b, 13b and 14b, the brightness between F1 and F2 detected by a detector) as the A value increases (as the axicon height decreases and the curvature increases) when the $\theta$ value is fixed, so the extended-depth-of-focus effects becomes prominent.

Meanwhile, referring to FIG. 9b and FIG. 12b, it can be confirmed that the extended-depth-of-focus effects increase as the $\theta$ value decreases while the A value is fixed.

Meanwhile, as shown in FIGS. 9a to 14b, it can be confirmed that not only the extended-depth-of-focus effects between F1 and F2, but also the extended-depth-of-focus effects between F2 and F3 depend upon the shape of the axicon portion 110. That is, various extended-depth-of-focus effects between a near distance and a distant distance may be obtained by setting the A value or the θ value of the axicon portion 110 according to the present invention.

Meanwhile, Meanwhile, the size of the central region 142, or $R_0$, which determines the size of the central region, affects the light intensity of an extended depth of focus. That is, referring to FIG. 11b, an extended depth of focus where the light intensity (height) is connected between F1 and F2 can be confirmed, and the light intensity height of the expanded region changes when the size of the central region(or $R_0$) is changed. For example, when $R_0$ is reduced, the light intensity in F3 shown in FIG. 11b becomes smaller, and when $R_0$ is increased, the light intensity in F3 shown in FIG. 11b becomes larger.

Although the present invention has been described with reference to embodiments shown in the drawings, the embodiments are provided as only exemplary examples, and those skilled in the art will understand that various modifications and equivalent other embodiments are possible therefrom. Therefore, the true technical protection scope of the present invention should be determined by the technical spirit of the appended claims.

**Claims**

1.  A multifocal lens having a central axicon portion, the multifocal lens comprising:

    a base lens forming a single focal point;
    a diffractive lens formed on the base lens, provided with a central region and a diffractive region surrounding the central region, and configured to disperse the single focal point into multiple focal points to form multiple focal points; and
    an axicon portion formed in the central region to protrude in a central axis direction of the base lens such that a depth of focus is expanded between the multiple focal points.

2.  The multifocal lens having a central axicon portion according to claim 1, wherein the diffractive lens and the axicon portion are formed on a front or back surface or both surfaces of the base lens.

3.  The multifocal lens having a central axicon portion according to claim 1, wherein a shape of the axicon portion is modeled as in a mathematical equation below:

<Mathematical equation>

$$z = -\sqrt{A^2 + \frac{r^2}{\tan^2\left(\frac{\theta}{2}\right)}}$$

where z indicates a distance from a vertex axis, which passes through a vertex of an axicon shape and is perpendicular to the central axis, to the axicon shape, A indicates a distance from the vertex axis to the vertex of the axicon shape, r indicates a radius that is a distance from the central axis to the axicon shape, and θ indicates an angle between two generators of the axicon shape.

4.  The multifocal lens having a central axicon portion according to claim 3, wherein an angle (θ) between both generators of the axicon shape is not 180°.

5.  The multifocal lens having a central axicon portion according to claim 3, wherein, as the distance A from the vertex axis to the vertex of the axicon shape increases in a state where the angle (θ) between both the generators of the axicon shape is fixed, extended-depth-of-focus effects increase.

6.  The multifocal lens having a central axicon portion according to claim 3, wherein, as the angle (θ) between both the generators of the axicon shape is reduced in a state where the distance A from the vertex axis to the vertex of the axicon shape is fixed, extended-depth-of-focus effects increase.

7.  The multifocal lens having a central axicon portion according to claim 5 or 6, wherein the distance A from the vertex axis to the vertex of the axicon shape or the angle (θ) between both the generators of the axicon shape is set such that extended-depth-of-focus effects increase in a distance between a near-distance focal point and an intermediate-distance focal point in a focal point generated along the axicon shape.

8.  The multifocal lens having a central axicon portion according to claim 3, wherein the multifocal lens has a concave shape when looking at a reference vertex q from the central axis when a sign of the z value becomes (+).

9.  The multifocal lens having a central axicon portion according to claim 3, wherein the distance A from the vertex axis to the vertex of the axicon shape or the angle (θ) between both the generators of the axicon shape is set such that extended-depth-of-focus effects increase in a distance between an intermediate-distance focal point and a distant-distance focal point in a focal point generated along the axicon shape when a sign of the z value becomes (+).

10. The multifocal lens having a central axicon portion according to claim 1, wherein a size of the central region affects light intensity of an extended depth of focus.

11. The multifocal lens having a central axicon portion according to claim 1, wherein heights of a plurality of steps in the diffractive region increase or decrease along a radial direction of a lens element.

12. The multifocal lens having a central axicon portion according to claim 1, wherein a height of a vertex of an axicon shape formed in the central region is equal to or smaller than a height of a step formed in a diffractive region.

13. The multifocal lens having a central axicon portion according to claim 1, wherein the central region and the diffractive region are attached to each other based on a certain boundary line.

【FIG. 1】

【FIG. 2】

【FIG. 3】

【FIG. 4】

【FIG. 5】

【FIG. 6】

【FIG. 7】

【FIG. 8】

【FIG. 9A】

【FIG. 9B】

【FIG. 10A】

【FIG. 10B】

【FIG. 11A】

【FIG. 11B】

【FIG. 12A】

【FIG. 12B】

【FIG. 13A】

【FIG. 13B】

【FIG. 14A】

【FIG. 14B】

## INTERNATIONAL SEARCH REPORT

International application No.

**PCT/KR2023/010165**

### A. CLASSIFICATION OF SUBJECT MATTER

**G02B 3/10**(2006.01)i; **G02B 5/00**(2006.01)i

According to International Patent Classification (IPC) or to both national classification and IPC

### B. FIELDS SEARCHED

Minimum documentation searched (classification system followed by classification symbols)

G02B 3/10(2006.01); A61B 3/00(2006.01); A61B 3/103(2006.01); A61F 2/16(2006.01); G02C 7/04(2006.01); G02C 7/06(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 초점심도(depth of focus, DOF), 회절 렌즈(diffraction lens), 다초점 렌즈(multifocal lens), 액시콘(axicon), 원추(cone), 회절(diffraction), 프레넬 렌즈(fresnel lens), 베셀빔(bessel beam)

### C. DOCUMENTS CONSIDERED TO BE RELEVANT

| Category* | Citation of document, with indication, where appropriate, of the relevant passages | Relevant to claim No. |
|---|---|---|
| Y | JP 2012-509152 A (ALCON INC.) 19 April 2012 (2012-04-19)<br>See paragraphs [0013]-[0014] and [0019]-[0021] and figures 2A-2C. | 1-2,10-13 |
| A | | 3-9 |
| Y | JP 2019-535445 A (NOVARTIS AG) 12 December 2019 (2019-12-12)<br>See paragraphs [0014]-[0018] and [0023]-[0030] and figures 1A-1B and 7-8. | 1-2,10-13 |
| A | US 2021-0321869 A1 (ALCON INC.) 21 October 2021 (2021-10-21)<br>See paragraphs [0031], [0056] and [0061] and figure 2B. | 1-13 |
| A | WO 2021-089178 A1 (VSY BIYOTEKNOLOJI VE ILAÇ SAN. A.S.) 14 May 2021 (2021-05-14)<br>See page 28, line 1 - page 29, line 4 and figure 3. | 1-13 |

☑ Further documents are listed in the continuation of Box C.   ☑ See patent family annex.

| | | |
|---|---|---|
| * | Special categories of cited documents: | "T" later document published after the international filing date or priority date and not in conflict with the application but cited to understand the principle or theory underlying the invention |
| "A" | document defining the general state of the art which is not considered to be of particular relevance | |
| "D" | document cited by the applicant in the international application | "X" document of particular relevance; the claimed invention cannot be considered novel or cannot be considered to involve an inventive step when the document is taken alone |
| "E" | earlier application or patent but published on or after the international filing date | |
| "L" | document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" | document referring to an oral disclosure, use, exhibition or other means | |
| "P" | document published prior to the international filing date but later than the priority date claimed | "&" document member of the same patent family |

| Date of the actual completion of the international search | Date of mailing of the international search report |
|---|---|
| **11 October 2023** | **12 October 2023** |

| Name and mailing address of the ISA/KR | Authorized officer |
|---|---|
| **Korean Intellectual Property Office**<br>**Government Complex-Daejeon Building 4, 189 Cheongsa-**<br>**ro, Seo-gu, Daejeon 35208** | |
| Facsimile No. **+82-42-481-8578** | Telephone No. |

Form PCT/ISA/210 (second sheet) (July 2022)

## INTERNATIONAL SEARCH REPORT

| International application No. |
| --- |
| **PCT/KR2023/010165** |

### C. DOCUMENTS CONSIDERED TO BE RELEVANT

| Category* | Citation of document, with indication, where appropriate, of the relevant passages | Relevant to claim No. |
| --- | --- | --- |
| A | KR 10-2011-0125652 A (THE ARIZONA BOARD OF REGENTS ON BEHALF OF THE UNIVERSITY OF ARIZONA) 21 November 2011 (2011-11-21)<br>See paragraphs [0023]-[0024] and figure 4. | 1-13 |

Form PCT/ISA/210 (second sheet) (July 2022)

## INTERNATIONAL SEARCH REPORT
### Information on patent family members

| International application No. |
| --- |
| **PCT/KR2023/010165** |

| Patent document cited in search report | | | Publication date (day/month/year) | Patent family member(s) | | | Publication date (day/month/year) |
| --- | --- | --- | --- | --- | --- | --- | --- |
| JP | 2012-509152 | A | 19 April 2012 | AU | 2017-369976 | A1 | 16 May 2019 |
| | | | | AU | 2022-291548 | A1 | 02 February 2023 |
| | | | | AU | 369976 | B2 | 13 October 2022 |
| | | | | CA | 3042203 | A1 | 07 June 2018 |
| | | | | CN | 110062899 | A | 26 July 2019 |
| | | | | CN | 110062899 | B | 28 May 2021 |
| | | | | CN | 113180887 | A | 30 July 2021 |
| | | | | EP | 3548936 | A1 | 09 October 2019 |
| | | | | JP | 2022-058843 | A | 12 April 2022 |
| | | | | JP | 7021213 | B2 | 16 February 2022 |
| | | | | US | 10842617 | B2 | 24 November 2020 |
| | | | | US | 11364112 | B2 | 21 June 2022 |
| | | | | US | 2018-0147052 | A1 | 31 May 2018 |
| | | | | US | 2021-0030532 | A1 | 04 February 2021 |
| | | | | US | 2022-0273424 | A1 | 01 September 2022 |
| | | | | WO | 2018-100452 | A1 | 07 June 2018 |
| JP | 2019-535445 | A | 12 December 2019 | AR | 076743 | A1 | 06 July 2011 |
| | | | | AU | 2009-316661 | A1 | 27 May 2010 |
| | | | | AU | 316661 | B2 | 14 November 2013 |
| | | | | BR | PI0920951 | A2 | 29 December 2015 |
| | | | | CA | 2741193 | A1 | 27 May 2010 |
| | | | | CA | 2741193 | C | 13 January 2015 |
| | | | | CN | 102223856 | A | 19 October 2011 |
| | | | | CN | 102223856 | B | 04 March 2015 |
| | | | | EP | 2349093 | A1 | 03 August 2011 |
| | | | | EP | 2349093 | A4 | 12 June 2013 |
| | | | | EP | 2349093 | B1 | 21 October 2015 |
| | | | | ES | 2556212 | T3 | 14 January 2016 |
| | | | | IL | 212634 | A | 31 March 2015 |
| | | | | JP | 5785093 | B2 | 24 September 2015 |
| | | | | KR | 10-1481964 | B1 | 14 January 2015 |
| | | | | KR | 10-2011-0086858 | A | 01 August 2011 |
| | | | | MX | 2011004729 | A | 30 May 2011 |
| | | | | NZ | 592572 | A | 25 October 2013 |
| | | | | RU | 2011124522 | A | 27 December 2012 |
| | | | | RU | 2526426 | C2 | 20 August 2014 |
| | | | | TW | 201026296 | A | 16 July 2010 |
| | | | | US | 2010-0131060 | A1 | 27 May 2010 |
| | | | | WO | 2010-059764 | A1 | 27 May 2010 |
| | | | | ZA | 201103275 | B | 25 July 2012 |
| US | 2021-0321869 | A1 | 21 October 2021 | AU | 2021-254864 | A1 | 22 September 2022 |
| | | | | CA | 3169531 | A1 | 21 October 2021 |
| | | | | CN | 115397307 | A | 25 November 2022 |
| | | | | EP | 4135562 | A1 | 22 February 2023 |
| | | | | JP | 2023-521855 | A | 25 May 2023 |
| | | | | WO | 2021-209866 | A1 | 21 October 2021 |
| WO | 2021-089178 | A1 | 14 May 2021 | AU | 2019-472967 | A1 | 19 May 2022 |
| | | | | BR | 112022008176 | A2 | 12 July 2022 |
| | | | | CA | 3155467 | A1 | 14 May 2021 |
| | | | | CN | 114651203 | A | 21 June 2022 |

Form PCT/ISA/210 (patent family annex) (July 2022)

**INTERNATIONAL SEARCH REPORT**
Information on patent family members

| International application No. |
| --- |
| **PCT/KR2023/010165** |

| Patent document cited in search report | | | Publication date (day/month/year) | Patent family member(s) | | | Publication date (day/month/year) |
| --- | --- | --- | --- | --- | --- | --- | --- |
| | | | | EP | 4055438 | A1 | 14 September 2022 |
| | | | | IL | 292431 | A | 01 June 2022 |
| | | | | JP | 2023-509811 | A | 10 March 2023 |
| | | | | US | 2022-0269110 | A1 | 25 August 2022 |
| KR | 10-2011-0125652 | A | 21 November 2011 | AU | 2010-213535 | A1 | 08 September 2011 |
| | | | | AU | 213535 | B2 | 09 July 2015 |
| | | | | BR | PI1008369 | A2 | 06 March 2018 |
| | | | | BR | PI1008369 | B1 | 29 October 2019 |
| | | | | CA | 2752164 | A1 | 19 August 2010 |
| | | | | CA | 2752164 | C | 30 May 2017 |
| | | | | CN | 102395906 | A | 28 March 2012 |
| | | | | CN | 102395906 | B | 28 May 2014 |
| | | | | EP | 2396683 | A2 | 21 December 2011 |
| | | | | EP | 2396683 | A4 | 02 April 2014 |
| | | | | EP | 2396683 | B1 | 08 April 2020 |
| | | | | EP | 3719544 | A1 | 07 October 2020 |
| | | | | ES | 2809181 | T3 | 03 March 2021 |
| | | | | IL | 214491 | A | 21 April 2016 |
| | | | | JP | 2012-517625 | A | 02 August 2012 |
| | | | | JP | 2016-189026 | A | 04 November 2016 |
| | | | | JP | 6042067 | B2 | 14 December 2016 |
| | | | | KR | 10-1727760 | B1 | 17 April 2017 |
| | | | | MX | 008529 | A | 24 October 2011 |
| | | | | MX | 2011008529 | A | 24 October 2011 |
| | | | | NZ | 594697 | A | 28 February 2014 |
| | | | | RU | 2011137403 | A | 20 March 2013 |
| | | | | RU | 2516035 | C2 | 20 May 2014 |
| | | | | SG | 10201402266 | XA | 30 October 2014 |
| | | | | SG | 173630 | A1 | 29 September 2011 |
| | | | | US | 10209533 | B2 | 19 February 2019 |
| | | | | US | 10725320 | B2 | 28 July 2020 |
| | | | | US | 11199725 | B2 | 14 December 2021 |
| | | | | US | 11693260 | B2 | 04 July 2023 |
| | | | | US | 2011-0292335 | A1 | 01 December 2011 |
| | | | | US | 2016-0341978 | A1 | 24 November 2016 |
| | | | | US | 2019-0339545 | A1 | 07 November 2019 |
| | | | | US | 2021-0003863 | A1 | 07 January 2021 |
| | | | | US | 2022-0197055 | A1 | 23 June 2022 |
| | | | | US | 9320594 | B2 | 26 April 2016 |
| | | | | WO | 2010-093975 | A2 | 19 August 2010 |
| | | | | WO | 2010-093975 | A3 | 02 December 2010 |

Form PCT/ISA/210 (patent family annex) (July 2022)